# EUROPEAN PATENT SPECIFICATION

(11) **EP 0 717 049 B1**
(45) Date of publication and mention of the grant of the patent: **01.04.1998**
(21) Application number: 94119902.8
(22) Date of filing: 16.12.1994
(51) Int. Cl.: C07K 14/81

(54) **Process for the extraction and purification of alfa1-antitrypsin (alfa1-pI) from cohn IV-1 fraction**
Verfahren zur Extraktion und Reinigung von Alpha-1-Antitrypsin (alpha 1-pI) aus Cohnischer Fraktion IV-1
Procédé pour l'extraction et la purification de l'alpha-1-antitrypsin (alpha 1-pI) à partir de la fraction IV-1 de Cohne

(30) Priority: 16.12.1994 IT FI930260
(43) Date of publication of application: 19.06.1996
(73) Proprietor: SCLAVO S.p.A., I-53100 Siena (IT); I.S.I. ISTITUTO SIEROVACCINOGENO ITALIANO S.P.A., Castelvecchio Pascoli (LU) (IT)
(72) Inventor: Arrighi, Silvana, 53010 Casciano di Murlo (IT); Bardotti, Angela, 53100 Siena (IT); Bucci, Enzo, 02015 Cittaducale (IT)
(74) Representative: Gervasi, Gemma, Dr.

(56) References cited:
- FR-A- 2 610 633
- BIOCHEMISTRY, vol. 13, no. 26, 1974 EASTON, PA US, pages 5439-5445, R PANNELL ET AL. 'Isolation and properties of human plasma alpha-1-Proteinase inhibitor'

## Description

### Field of the invention

The present invention relates to a process for the extraction and following purification of alfa1-antitrypsin (alfa1-pI) from Cohn IV-1 Fraction obtained by cold fractionation of plasma with alcohol according to the method of Cohn.

In particular, the present invention relates to a process for extraction and purification comprising the following steps:
a) solubilization of starting paste (IV-1 Fraction) and fractioned precipitation;
b) hydrophobic interaction chromatography;
c) viral inactivation; and
d) final operations.

### State of the art

Alfa1-antitrypsin (alfa1-pI) protein is a proteases plasmatic inhibitor and it is used for the treatment of pulmonary emphysema in subjects with alfa1-pI congenital or acquired deficiency.

US 4,379,087 describes a process for the extraction of alfa1-pI comprising the following steps:
I) solubilization of IV-1 Fraction and precipitation of contaminants by addition of polyethylene glycol (10-15%);
II) anionic exchange chromatography with absorption of proteins on resin and following selective elution of the product.

By means of such process it is possible to obtain a product with specific activity of 0.6 mg alfa-pI/mg total proteins and a yield corresponding to 50% of the IV-1 Fraction.

EP-A-282363 describes a process comprising the following steps:
i) suitable dilution of II + III supernatant (according to the method of Cohn) (adjustments of pH and ionic strength);
ii) anionic exchange chromatography;
iii) concentration and dialysis;
iv) gel-filtration chromatography.

Thanks to this method it is possible to obtain a product with specific activity of 0.8 mg alfa1-pI/mg total proteins.

US 4,684,723 describes a process comprising the formation of two immiscible liquid phases, constituted by a solution of inorganic salts and a solution of polyetilene glycol respectively; alfa1-pI is preferably diluted in one of these two phases thus being separated from its contaminants.

C.G. Glasser et al., 1982, An. Biochem., 124, 364-371, describes a process of purification comprising a precipitation with ammonium sulphate in the presence of a reducing substance such as dithiotreitol, which denatures proteins with disulfate bridges, followed by anion exchange chromatography.

R. Panell, et al., 1974, Biochem., 13, 5439-5445, describes a process providing the removal of albumin by adsorption on Dextran Blue-Sepharose, precipitation with ammonium sulfate and anionic exchange chromatography.

### Detailed description of the invention

It has now surprisingly been found that is easily possible to separate the alfa1-pI by the other components of Cohn IV-1 Fraction by passing such Fraction (previously treated in order to eliminate part of the contaminant proteins) on a hydrophobic interaction resin, thus obtaining a product of high purity. The present invention enables the extraction and following purification of alfa1-pI by means of a new process comprising the following steps:
a) solubilization of starting paste constituted by Cohn IV-1 Fraction and fractioned precipitation;
b) hydrophobic interaction chromatography;
c) viral inactivation; and
d) final operations comprising diafiltration, protein concentration and optionally aseptical dispensation into vials and lyophilization.

The process according to the present invention enables the recovery of about 40% of alpha-pI present in IV-1 Fraction with a purity higher than 0.6 mg alfa1-pI/mg total proteins.

Hereinafter it is described a preferred embodiment of the process according to the invention comprising the above mentioned steps a) - d):
a) solubilizing an amount of starting paste obtained by cold fractionation of plasma with alcohol according to the method of Cohn, cooling the obtained solution and adding ammonium sulfate, centrifuging the resulting suspension and removing the precipitate containing the contaminant proteins and then recovering the solution containing alfa1-pI; the final solution may either be frozen or immediately subjected to the chromatographic purification of step b);
b) loading the solution obtained from step a) onto a chromatographic column, which contains a hydrophobic interaction resin previously conditioned with a suitable TC buffer (conditioning buffer), and recovering the unbound alfa1-pI, then washing the column with the above buffer and recovering the alfa1-pI which was weakly bound to the resin;
c) dialyzing and concentrating the solution, containing the alfa1-pI, obtained from step b); such solution may either be frozen or immediately subjected to a process of viral inactivation;
d) diafiltering and concentrating the solution subjected to viral inactivation, obtained from step c), up to the desired protein concentration, and then aseptically dispensing into vials and lyophilizing the thus obtained product.

According to a further preferred embodiment, the process according to the invention is characterized as follows.

### Step a):

Solubilizing the IV-1 Fraction in a ratio of 1/6 - 1/10 (preferably 1/8), up to 300-500 litres (preferably 400) with 0.07-0.1M (preferably 0.1M) Tris-HCl buffer at pH 7.8-8.5 (preferably 8.2) under stirring, in a costant-temperature chamber maintained at 30-41°C (preferably 40°C); stirring the solution for 0.30-1.30 hours (preferably 1 hour) once reached the desired range of temperature.

Cooling the solution at 2°-6°C (preferably 4°C) and gradually adding 243-351 g/l (preferably 313 g/l) of ammonium sulfate, at 2-6°C (preferably 4°C), in order to obtain a solution of 40-55% (preferably 50%) of saturation. Maintaining the solution under stirring at the same temperature for 18-30 hours (preferably 24 hours).

Centrifuging the obtained suspension and removing the obtained precipitate containing the contaminant proteins. The final solution contains 60-80% of the alfa1-pI which was present in the starting paste, with a specific activity of 0.1-0.35 mg alfa1-pI/mg total proteins and then resulting in a purification factor of 2.5-8.5.

### Step b):

Adjusting the solution obtained from step a) up to pH 6.8-7.2 (preferably 7.0) with 1N HCl or 1N CH₃COOH, and then filtering said solution under sterile conditions (preferably up to 0.45 micron). Then, loading such solution on a chromatographic column, with a load of 10-25 mg (preferably 20 mg) of total proteins/ml of resin, such column containing 40-70 litres (preferably 50 litres) of hydrophobic interaction resin [for example, Phenyl Sepharose CL-4B® (Pharmacia), Phenyl Sepharose Fast Flow high and low subst., Phenyl Sepharose High Performance (Pharmacia), Fractogel EMD Phenyl (S)® (E.Merck) or Silica PAE-1000L® (Amicon)] conditioned with TC buffer: 0.07-0.15M (preferably 0.1M) monobasic phosphate buffer, 1.9-2.1M (preferably 2.0M) ammonium sulfate, pH 6.9-7.1 (preferably 7.0) at a flow rate of 30-70 cm/h (preferably 50 cm/h), costant in all several chromatographic steps.

The unbound alfa1-pI retained in the void volume and the alfa1-pI weakly bound to the resin are recovered by passages of 3-7 column beds (preferably 5 column beds) of TC buffer.

The chromatography of step b) gives a yield of 55-75%.

### Step c):

Performing the viral inactivation according to techniques known in the art.

### Step d):

Diafiltering the solution against a 15-25mM (preferably 20mM) phosphate buffer containing 0.12-0.14 sodium chloride (preferably 0.13M), pH 6.9-7.1 (preferably 7.0).

The thus obtained alfa1-pI protein is concentrated up to the desired values, filtered under sterile conditions, aseptically dispensed into vials and lyophilized according to a method known in the art. The desired protein concentration is preferably in the form of 10, 15 or 25 mg/ml alfa1-pI.

The final product has a specific activity of 0.6-0.8 mg alfa1-pI/mg total proteins. Such process, therefore, enables the obtainment of a total purification factor of about 18-20.

Further advantages and features of the process according to the invention will be more properly understood in the light of the following example.

### EXAMPLE

a) 50 Kg of IV-1 Fraction, obtained by cold fractionation of plasma with alcohol according to the method of Cohn, were solubilized and adjusted till having 400 litres with 0.1M Tris-HCl pH 8.2 buffer, under continuos stirring, in a costant-temperature chamber maintained at 40°C.
   After reacing 40°C, the mixture was maintained under stirring for 1 hour.
   The obtained solution was cooled to 4°C; then 125 Kg of ammonium sulfate were gradually added, under continuous stirring, in order to obtain a solution with 50% saturation. Said solution was left under stirring at the same temperature for 24 hours.
   The obtained suspension was centrifuged and the obtained precipitate, containing contaminant proteins, was removed.
   The solution contained about 70% of alfa1-pI which was present in the starting paste, with a specific activity of about 0.2 mg alfa1-pI/mg total proteins thus resulting in a purification factor of about 5.
b) The solution obtained from step a) was adjusted to pH 7.0 with 1N HCl and then aseptically filtered till reaching 0.45 micron. The above solution was loaded onto a chromatographic column, with a load of 20 mg total proteins/ml resin. Said column contained 50 litres of hydrophobic interaction resin Phenyl Sepharose CL-4B® (Pharmacia), conditioned in TC buffer: 0.1M monobasic phosphate buffer, 2.0M ammonium sulfate, pH 7.0 and a flow rate of 50 cm/h, costant in all chromatographic steps.
   After loading the solution, the unbound alfa1-pI and the alfa1-pI weakly bound to the resin were recovered collecting the non-adsorbed part and successively washing the resin by passing 5 column beds of conditioning buffer. Then the contaminants were eluted from resin washing with 5 column beds with each of the following solutions:
   1) 0.1M pH 7.0 phosphate buffer;
   2) distilled water;
   3) 20% ethanol.

   The described chromatography gives a yield of 65%.
c) The solution, containing alfa1-pI, obtained from step b) in a 800 litres volume, was dialized and concentrated and then subjected to viral inactivation by liquid phase pasteurization for 10 hours at 60±1°C in the presence of suitable stabilizers.
d) The solution, after viral inactivation treatment performed in step c) was diafiltered against 20mM phosphate buffer, containing 0.13M NaCl, pH 7.0 and it was concentrated till reaching the proteic concentration of 25 mg/ml alfa1-pI, filtered under sterile conditions and then aseptically dispensed into vials and lyophilized.
   The final product had a specific activity of 0.7 mg alfa1-pI/mg total proteins. Therefore it was obtained a total purification factor about 18 times bigger than the IV-1 Fraction used as starting material.

## Claims

1. A process for the extraction and purification of alfa1-pI comprising the following steps:
(a) solubilization of starting paste (IV-1 Fraction) and fractioned precipitation;
(b) hydrophobic interaction chromatography;
(c) viral inactivation; and
(d) final operations comprising diafiltration, protein concentration and aseptical dispensation into vials and lyophilization.

2. A process for the extraction and purification of alfa1-pI according to claim 1 comprising the following steps:
(a) solubilizing IV-1 Fraction, obtained by cold fractionation of plasma with alcohol according to the method of Cohn, cooling and adding ammonium sulfate to form a suspension, centrifuging said suspension and removing a precipitate containing contaminant proteins and then recovering a solution containing alfa1-pI;
(b) loading the solution obtained from step (a) onto a chromatographic column, which contains a hydrophobic interaction resin previously conditioned with a suitable conditioning buffer, recovering the unbound alfa1-pI, and then washing the column with said buffer and recovering by collecting the alfa1-pI weakly bound to the resin, in order to form a solution containing the recovered alfa1-pI;
(c) dialyzing and concentrating the solution containing the alfa1-pI obtained from step (b); said solution may either be frozen or immediately subjected to a process of viral inactivation;
(d) diafiltering and concentrating the solution subjected to viral inactivation obtained from step (c) in order to form a final product, and then aseptically dispensing into vials and lyophilizing said product.

3. A process for the extraction and purification of alfa1-pI according to claim 2 comprising the following steps:
(a) solubilizing IV-1 Fraction, under continuous stirring, said fraction being obtained by cold fractionation of plasma with alcohol according to the method of Cohn, in a ratio of 1/6 - 1/10, till having 300-500 litres with 0.07-0.15M Tris-HCl buffer at pH 7.8-8.5, in a costant-temperature chamber maintained at 30-41°C; after reaching said temperature, stirring for 0.30-1.30 hours, cooling at 2°-6°C and gradually adding 243-351 g/l ammonium sulfate, in order to obtain a 40-55% solution, then leaving said solution under stirring at 2°-6°C for 18-30 hours giving a suspension; centrifuging said suspension and removing a precipitate containing contaminant proteins and then recovering a solution containing alfa1-pI;
(b) adjusting the solution containing alfa1-pI obtained from step (a) up to pH 6.8-7.2 with 1N HCl or 1N CH₃COOH, and then filtering said solution under sterile conditions, loading it onto a chromatographic column, with a load of 10-25 mg of total proteins/ml of resin, said column containing 40-70 litres of hydrophobic interaction resin, said resin being selected from the group consisting of Phenyl Sepharose CL-4B® (Pharmacia), Phenyl Sepharose Fast Flow high and low subst., Phenyl Sepharose High Performance (Pharmacia), Fractogel EMD Phenyl (S)® (E.Merck) or Silica PAE-1000L® (Amicon)] and conditioned with a conditioning buffer consisting of 0.07-0.15M monobasic sodium phosphate, 1.9-2.1M ammonium sulfate, pH 6.9-7.1 at a flow rate of 30-70 cm/h, said flow rate being costant in all chromatographic steps; recovering the unbound alfa1-pI retained in the void volume and further recovering the alfa1-pI weakly bound to the resin by passages of 3-7 column beds of said conditioning buffer, in order to form a solution containing the recovered alfa1-pI;
(c) dialyzing and concentrating the solution, containing the alfa1-pI, obtained from step (b); said solution may either be frozen or immediately subjected to a process of viral inactivation;
(d) diafiltering the solution obtained from step (c) against a 15-25mM phosphate buffer containing 0.12-0.14 sodium chloride, at pH 6.9-7.1, then concentrating said solution till reaching the values of 10, 15 or 25 mg/ml alfa1-pI, and then aseptically dispensing into vials and lyophilizing said solution.

4. The process according to claim 3, characterized in that in step (a) IV-1 Fraction is solubilized in a ratio of 1/8 till having 400 litres with 0.1M Tris-HCl buffer at pH 8.2, maintaining the costant-temperature chamber at 40°C and stirring for 1 hour, adding ammonium sulfate up to 313 g/l in order to form a solution with 50% saturation, and leaving said solution under stirring for 24 hours.

5. The process according to claim 3, characterized in that in step (b) pH is maintained at 7.0, 20 mg total proteins/ml resin are loaded onto a chromatographic column, said column containing 50 litres of resin and being conditioned with a conditioning buffer consisting of 0.1M monobasic sodium phosphate, 2.0M ammonium sulfate, pH 7.0 at a flow rate of 50 cm/h; said resin is then washed with 5 column beds of said conditioning buffer.

6. The process according to claim 3, characterized in that in step (d) the solution obtained from step (c) is diafiltered against a 20mM phosphate buffer containing 0.13 sodium chloride at pH 7.0.

## Patentansprüche

1. Verfahren zur Extraktion und Reinigung von alpha1-pI, umfassend die folgenden Schritte:
(a) Solubilisierung der Ausgangspaste (IV-1-Fraktion) und fraktionierte Ausfällung;
(b) hydrophobe Wechselwirkungschromatographie;
(c) Virusinaktivierung; und
(d) Endarbeitsgänge, umfassend Diafiltration, Proteinkonzentrierung und aseptische Verteilung in Phiolen und Gefriertrocknung.

2. Verfahren zur Extraktion und Reinigung von alpha1-pI gemäß Anspruch 1, umfassend die folgenden Schritte:
(a) Solubilisierung der IV-1-Fraktion, erhalten durch kalte Fraktionierung von Plasma mit Alkohol gemäß Cohn'schem Verfahren, Abkühlen und Zugabe von Ammoniumsulfat zur Bildung einer Suspension, Zentrifugieren der Suspension und Entfernen eines Niederschlags, enthaltend verunreinigende Proteine, und anschließende Gewinnung einer alpha1-pI-haltigen Lösung;
(b) Aufbringen der aus Schritt (a) erhaltenen Lösung auf eine Chromatographiesäule, die ein hydrophobes Wechselwirkungsharz enthält, das zuvor mit einem geeigneten Konditionierungspuffer konditioniert wurde, Gewinnung des ungebundenen alpha1-pI und anschließendes Waschen der Säule mit dem Puffer und Gewinnung durch Auffangen des schwach an das Harz gebundenen alpha1-pI, um eine Lösung zu bilden, die das gewonnene alpha1-pI enthält;
(c) Dialysieren und Konzentrieren der Lösung, die das aus Schritt (b) erhaltene alpha1-pI enthält, wobei die Lösung entweder gefroren oder direkt einem Virusinaktivierungsverfahren unterzogen werden kann;
(d) Diafiltration und Konzentrieren der aus Schritt (c) erhaltenen und einer Virusinaktivierung unterzogenen Lösung, um ein Endprodukt zu bilden, und anschließendes aseptisches Verteilen des Produkts in Phiolen und Gefriertrocknung.

3. Verfahren zur Extraktion und Reinigung von alpha1-pI gemäß Anspruch 2, umfassend die folgenden Schritte:
(a) Solubilisierung der IV-1-Fraktion unter kontinuierlichen Rühren, wobei die Fraktion durch kalte Fraktionierung von Plasma mit Alkohol gemäß Cohn'schem Verfahren erhalten wurde, in einem verhältnis von 1/6 bis 1/10, bis man 300 bis 500 ℓ mit 0,07 bis 0,15 M Tris-HCl-Puffer bei pH 7,8 bis 8,5 hat, in einer auf einer konstanten Temperatur von 30 bis 41°C gehaltenen Kammer; nach Erreichen der Temperatur Rühren für 0,30 bis 1,30 h, Abkühlen auf 2 bis 6°C und allmähliche Zugabe von 243 bis 351 g/ℓ Ammoniumsulfat, um eine 40 bis 55%ige Lösung zu erhalten, dann Stehenlassen der Lösung unter Rühren bei 2 bis 6°C für 18 bis 30 h, so daß sie eine Suspension ergibt; Zentrifugieren der Suspension und Entfernen eines Niederschlags, der verunreinigenden Proteine enthält, und anschließende Gewinnung einer alpha1-pI-haltigen Lösung;
(b) Einstellung der aus Schritt (a) erhaltenen alpha1-pI-haltigen Lösung auf einen pH von 6,8 bis 7,2 mit 1 N HCl oder 1 N CH₃COOH und anschließendes Filtrieren der Lösung unter sterilen Bedingungen, Aufbringen auf eine Chromatographiesäule bei einer Beladung von 10 bis 25 mg Gesamtproteine/ml Harz, wobei die Säule 40 bis 70 ℓ eines hydrophoben Wechselwirkungsharzes enthält, wobei das Harz ausgewählt ist aus Phenyl Sepharose CL-4B® (Pharmacia), Phenyl Sepharose Fast Flow hoch- und niedersubstituiert, Phenyl Sepharose High Performance (Pharmacia), Fractogel EMD Phenyl (S)® (E. Merck) und Silica PAE-1000L® (Amicon) und mit einem Konditionierungspuffer, bestehend aus 0,07 bis 0,15 M einbasigem Natriumphosphat und 1,9 bis 2,1 M Ammoniumsulfat, bei pH 6,9 bis 7,1 bei einer Laufgeschwindigkeit von 30 bis 70 cm/h konditioniert wurde, wobei die Laufgeschwindigkeit in allen Chromatographieschritten konstant ist; Gewinnung des im Leervolumen zurückbehaltenen ungebundenen alpha1-pI und weiterhin Gewinnung des schwach an das Harz gebundenen alpha1-pI durch Durchlaufen von 3 bis 7 Säulenbetten des Konditionierungspuffers, um eine Lösung zu erhalten, die das gewonnene alpha1-pI enthält;
(c) Dialysieren und Konzentrieren der aus Schritt (b) erhaltenen alpha1-pI-haltigen Lösung, wobei die Lösung entweder gefroren oder direkt einem Virusinaktivierungsverfahren unterzogen werden kann;
(d) Diafiltration der aus Schritt (c) erhaltenen Lösung gegen einen 0,12 bis 0,14 Natriumchlorid-haltigen 15 bis 25 mM Phosphatpuffer bei pH 6,9 bis 7,1, anschließendes Konzentrieren der Lösung bis zum Erreichen der Werte von 10, 15 oder 25 mg/ml alpha1-pI und anschließendes aseptisches Verteilen in Violen und Gefriertrocknen der Lösung.

4. Verfahren gemäß Anspruch 3,
dadurch **gekennzeichnet,** daß
in Schritt (a) die IV-1-Fraktion mit 0,1 M Tris-HCl-Puffer bei pH 8,2 in einem Verhältnis von 1/8 solubilisiert wird, bis man 400 l hat, wobei die Kammer bei konstanter Temperatur auf 40°C gehalten wird und man für 1 h rührt, Ammoniumsulfat bis zu 313 g/l hinzugegeben wird, um eine Lösung mit 50%iger Sättigung zu erhalten, und die Lösung unter Rühren für 24 h stehengelassen wird.

5. Verfahren gemäß Anspruch 3,
dadurch **gekennzeichnet,** daß
in Schritt (b) der pH auf 7,0 gehalten wird, 20 mg Gesamtproteine/ml Harz auf eine Chromatographiesäule aufgebracht werden, wobei die Säule 50 l des Harzes enthält und mit einem Konditionierungspuffer, der aus 0,1 M einbasigem Natriumphosphat und 2,0 M Ammoniumsulfat besteht, bei pH 7,0 bei einer Laufgeschwindigkeit von 50 cm/h konditioniert wurde, wobei das Harz anschließend mit 5 Säulenbetten des Konditionierungspuffers gewaschen wird.

6. Verfahren gemäß Anspruch 3,
dadurch **gekennzeichnet,** daß
in Schritt (d) die aus Schritt (c) erhaltene Lösung gegen einen 20 mM Phosphatpuffer diafiltriert wird, der 0,13 Natriumchlorid bei pH 7,0 enthält.

## Revendications

1. Procédé d'extraction et de purification de l'alpha-1-antitrypsine (alpha-1-pI) comprenant les étapes suivantes :
(a) solubilisation de la pâte de départ (Fraction IV-1) et précipitation fractionnée ;
(b) chromatographie par interaction hydrophobe ;
(c) inactivation virale ;
(d) opérations finales comprenant la diafiltration, la concentration des protéines, la répartition aseptique en flacons, et la lyophilisation.

2. Procédé d'extraction et de purification de l'alpha-1-pI selon la revendication 1, comprenant les étapes suivantes :
(a) solubilisation de la Fraction IV-1 obtenue par fractionnement à froid du plasma avec de l'alcool, selon la procédure de Cohn, refroidissement et addition de sulfate d'ammonium pour former une suspension, centrifugation de ladite suspension et élimination du précipité contenant des protéines contaminantes, puis récupération d'une solution contenant l'alpha-1-pI ;
(b) chargement de la solution obtenue à l'étape (a) sur une colonne de chromatographie contenant une résine à interaction hydrophobe, conditionnée au préalable à l'aide d'un tampon conditionnant approprié, récupération de l'alpha-1-pI non liée, puis lavage de la colonne avec ledit tampon, et récupération en collectant l'alpha-1-pI faiblement liée à la résine, pour former une solution contenant l'alpha-1-pI récupérée ;
(c) dialyse et concentration de la solution contenant l'alpha-1-pI obtenue à l'étape (b) ; ladite solution peut être soit congelée, soit soumise immédiatement à une procédure d'inactivation virale ;
(d) diafiltration et concentration de la solution soumise à l'inactivation virale obtenue à l'étape (c) afin de former un produit final, puis répartition aseptique en flacons, et lyophilisation dudit produit.

3. Procédé d'extraction et de purification de l'alpha-1-pI selon la revendication 2, comprenant les étapes suivantes :
(a) solubilisation de la Fraction IV-1 sous agitation continue, ladite fraction étant obtenue par fractionnement à froid du plasma avec de l'alcool, selon la procédure de Cohn, dans un ratio compris entre 1/6 et 1/10, jusqu'à obtention de 300 à 500 litres, avec du tampon Tris-HCl 0,07-0,15M à pH 7,8-8,5, dans une chambre à température constante maintenue entre 30 et 41°C ; une fois ladite température atteinte, agitation pendant 0,5 à 1,5 heure, refroidissement entre 2 et 6°C et addition graduelle de sulfate d'ammonium à raison de 243 à 351 g/l pour obtenir une solution à 40 à 55 %, puis maintien de ladite solution sous agitation entre 2 et 6°C pendant 18 à 30 heures pour obtenir une suspension ; centrifugation de ladite suspension et élimination d'un précipité contenant des protéines contaminantes, puis récupération d'une solution contenant de l'alpha-1-pI ;
(b) ajustage de la solution contenant l'alpha-1-pI obtenue à l'étape (a) à pH 6,8-7,2 par HCl 1N ou CH₃COOH 1N, puis filtration de ladite solution dans des conditions stériles, chargement de celle-ci sur une colonne de chromatographie à raison de 10 à 25 mg de protéines totales pour 100 ml de résine, ladite colonne contenant 40 à 70 litres d'une résine à interaction hydrophobe, ladite résine étant choisis au sein du groupe comprenant la Phényl Sepharose CL-4B ® (Pharmacia) ; la Phényl Sepharose Fast Flow high et low subst. ; la Phényl Sepharose High Performance (Pharmacia) ; la Fractogel EMD Phényl (S) ® (E. Merck) ou la Silica PAE-1000D ® (Amicon), et conditionnée à l'aide d'un tampon conditionnant consistant en phosphate de sodium monobasique 0,07-0,15M, sulfate d'ammonium 1,9-2,1M, à pH 6,9-7,1, à un débit de 30 à 70 cm/heure, ledit débit étant constant dans toutes les étapes de chromatographie ; récupération de l'alpha-1-pI non liée retenue dans le volume mort, puis récupération de l'alpha-1-pI faiblement liée à la résine, par des passages dudit tampon conditionnant, à raison de 3 à 7 lits de garnissage de la colonne, pour donner une solution contenant l'alpha-1-pI récupérée ;
(c) dialyse et concentration de la solution contenant l'alpha-1-pI obtenue à l'étape (b) ; ladite solution pouvant être soit congelée, soit soumise immédiatement à une procédure d'inactivation virale ;
(d) diafiltration de la solution obtenue à l'étape (c) en présence de tampon phosphate 15-25µM contenant du chlorure de sodium 0,12 à 0,14N, à pH 6,9-7,1, puis concentration de ladite solution jusqu'à obtention des valeurs de 10, 15 ou 25 mg/ml en alpha-1-pI, et ensuite répartition aseptique en flacons, et lyophilisation de ladite solution.

4. Procédé selon la revendication 3, caractérisé en ce que dans l'étape (a), la Fraction IV-1 est solubilisée à un ratio de 1/8 jusqu'à obtention de 400 litres, avec du tampon de Tris-HCl 0,07-0,15M, à pH 8,2, en maintenant à 40°C la chambre à température constante, et en ajoutant du sulfate d'ammonium jusqu'à 313 g/litre afin de former une solution saturée à 50 %, et ladite solution est laissée sous agitation pendant 24 heures.

5. Procédé selon la revendication 3, caractérisé en ce que, à l'étape (b), le pH est maintenu à 7,0, le chargement est fait sur une colonne de chromatographie contenant 50 litres de résine, à raison de 20 mg de protéines totales par ml, ladite résine étant conditionnée à l'aide d'un tampon conditionnant consistant en phosphate de sodium monobasique 0,1M, en sulfate d'ammonium 2,0M, à pH 7,0, à un débit de 50 cm/heure ; ladite résine étant ensuite lavée avec ledit tampon conditionnant à raison de 5 lits de garnissage de colonne.

6. Procédé selon la revendication 3, caractérisé en ce que, à l'étape (d), la solution obtenue à l'étape (c) est diafiltrée en présence de tampon phosphate 20µM contenant du chlorure de sodium 0,13N, à pH 7,0.
